# EUROPÄISCHE PATENTSCHRIFT

(11) **EP 1 252 136 B1**
(45) Veröffentlichungstag und Bekanntmachung des Hinweises auf die Patenterteilung: **23.06.2004**
(21) Anmeldenummer: 01905678.7
(22) Anmeldetag: 24.01.2001
(51) Int. Cl.: C07C 275/60, C07C 271/12, C08K 5/205, C08K 5/21

(54) **DIETHYLOCTANDIOLDICARBAMATE UND DIETHYLOCTANDIOLDIALLOPHANATE, VERFAHREN ZU IHRER HERSTELLUNG UND IHRE VERWENDUNG**
DIETHYLOCTANDIOLDICARBAMATES AND DIETHYLOCTANDIOLDIALLOPHANATES, METHOD FOR THE PRODUCTION AND USE THEREOF
DICARBAMATES DE DIETHYL-OCTANE-DIOL ET DIALLOPHANATES DE DIETHYL-OCTANE-DIOL, LEURS PROCEDES DE PRODUCTION ET LEUR UTILISATION

(30) Priorität: 02.02.2000 DE 10004498
(43) Veröffentlichungstag der Anmeldung: 30.10.2002
(73) Patentinhaber: BASF Coatings AG, 48165 Münster (DE)
(72) Erfinder: RINK, Heinz-Peter, 48153 Münster (DE); HENKELMANN, Jochem, 68165 Mannheim (DE); JUNG, Werner-Alfons, 59387 Ascheberg (DE); HARRIS, Paul, West Bloomfield, MI 48322 (US); RAMESH, Swaminathan, Canton, MI 48187 (US)
(74) Vertreter: Fitzner, Uwe, Dr.
(86) Internationale Anmeldenummer: PCT/EP2001/000729
(87) Internationale Veröffentlichungsnummer: WO 2001/056978

(56) Entgegenhaltungen:
- EP-A- 0 850 986
- DE-A- 4 432 897

## Beschreibung

Die vorliegende Erfindung betrifft neue stellungsisomere Diethyloctandioldicarbamate und Diethyloctandioldiallophanate. Des weiteren betrifft die vorliegende Erfindung neue Verfahren zur Herstellung dieser neuen Verbindungen. Außerdem betrifft die vorliegende Erfindung die Verwendung dieser neuen Verbindungen als Synthesebausteine und als Bestandteile von Klebstoffen, Dichtungsmassen und Beschichtungsstoffen. Darüber hinaus betrifft die vorliegende Erfindung neue Klebstoffe, Dichtungsmassen und Beschichtungsstoff, die die neuen Verbindungen enthalten. Ferner betrifft die vorliegende Erfindung neue Klebschichten, Dichtungen und Beschichtungen, die mit Hilfe der neuen Klebstoffe, Dichtungsmassen um Beschichtungsstoff hergestellt werden können. Nicht zuletzt betrifft die vorliegende Erfindung neue grundierte und ungrundierte Substrate, die die neuen Klebschichten, Dichtungen und/oder Beschichtungen aufweisen.

Aus den Patentschriften US-A-5,474,811, US-A-5,356,669, US-A-5,605,965, WO 94/10211, WO 94/10212, WO 94/10213, EP-A-0 594 068, EP-A-0 594 071 oder EP-A-0 594 142 sind thermisch härtbare Beschichtungsstoffe bekannt, welche Bindemittel (vgl. hierzu Römpp Lexikon Lacke und Druckfarben, Georg Thieme Verlag, Stuttgart, York, 1998, »Bindemittel«, Seiten 73 und 74) mit mindestens einer seitenständigen und/oder endständigen Carbamatgruppe der Formel:

**-O-(CO)-NH**_{**2**}

und mindestens einem Vernetzungsmittel, enthaltend mindestens 2 funktionelle Gruppen, welche mit der Carbamatgruppe Vernetzungsreaktionen eingehen, enthalten. Diese bekannten Beschichtungsmittel liefern Klarlacke, welche eine außerordentlich hohe Kratzfestigkeit und Etchbeständigkeit aufweisen. Als Vernetzungsmittel werden vor allem hochveretherte Melaminformaldehydharze angewandt. Die Vemetzung selbst ist säurekatalysiert, wobei als Katalysatoren bevorzugt starke Protonensäuren, insbesondere Sulfonsäuren, welche i. d. R. mit Aminen blockiert sind, verwendet werden.

Des weiteren sind aus der europäischen Patentanmeldung 97122649.3-2102 thermisch härtbare Beschichtungsstoffe bekannt, die carbamatfunktionalisierte (vgl. die vorstehende Formel) Aminoplastharze enthalten. Diese Verbindungen werden als Vernetzungsmittel für nicht funktionalisierte Aminoplastharze oder für Bindemittel mit seitenständigen carbamatreaktiven funktionellen Gruppen eingesetzt. Die Beschichtungsstoffe liefern ebenfalls Beschichtungen, die sehr gute anwendungstechnische Eigenschaften aufweisen.

Diese bekannten, stickstoffreichen Beschichtungsstoffe weisen häufig eine hohe Viskosität auf, die der Applikation und dem Verlauf der Beschichtungsstoffe abträglich ist. Es besteht daher das Bedürfnis, die Viskosität der Beschichtungsstoffe zu erniedrigen, ohne daß der Feststoffgehalt der Beschichtungsstoffe abgesenkt werden muß. Außerdem besteht das Bedürfnis, den Anteil der komplementären reaktiven funktionellen Gruppen, insbesondere den Anteil der Carbamatgruppen oder analoger Gruppen, die für die thermische Vernetzung notwendig sind, weiter zu erhöhen. Hierbei soll indes keine weitere Erhöhung der Hydrophilie der Beschichtungsstoffe und der Beschichtungen eintreten, damit das bereits hohe Niveau der Säurebeständigkeit und der Feuchtigkeitbeständigkeit nicht abgesenkt wird.

Hierbei ist unter der Eigenschaft hydrophil die konstitutionelle Eigenschaft eines Moleküls oder einer funktionellen Gruppe zu verstehen, in die wäßrige Phase einzudringen oder darin zu verbleiben. Ergänzend wird aufRömpp Lexikon Lacke und Druckfarben, Georg Thieme Verlag, Stuttgart, New York, 1998, »Hydrophilie«, »Hydrophobie«, Seiten 294 und 295, verwiesen.

Die Aufgabe der vorliegenden Erfindung ist es, neue carbamatgruppenhaltige und/oder allophanatgruppenhaltige Klebstoffe, Dichtungsmassen und Beschichtungsstoffe bereitzustellen, die thermisch oder thermisch und mit aktinischer Strahlung (Dual Cure) gehärtet werden können und die die Nachteile des Standes der Technik nicht mehr länger aufweisen, sondern bei einem hohen Feststoffgehalt und einem hohen Anteil an stickstoffhaltigen, thermisch vernetzenden komplementären reaktiven funktionellen Gruppen eine vergleichsweise niedrige Viskosität aufweisen und Klebstoffe, Dichtungsmassen und Beschichtungen liefern, die eine hohe Säurebeständigkeit und Feuchtigkeitsbeständigkeit haben.

Außerdem war es die Aufgabe der vorliegenden Erfindung, neue Verbindungen bereitzustellen, die als Bestandteile von thermisch oder thermisch und mit aktinischer Strahlung härtbaren Klebstoffen, Dichtungsmassen und Beschichtungsstoffen in hohem Maße in Betracht kommen.

Des weiteren war es die Aufgabe der vorliegenden Erfindung, neue Verbindungen bereitzustellen, die als Synthesebausteine in der hochmolekulare und niedermolekularen organischen Chemie und der metallorganischen Chemie fungieren können.

Demgemäß wurden die neuen stellungsisomeren Diethyloctandioldicarbamate und Diethyloctandioldiallophanate gefunden, die im folgenden zusammenfassend als "erfindungsgemäße Verbindungen" bezeichnet werden.

Außerdem wurden neue Verfahren zur Herstellung der erfindungsgemäßen Verbindungen gefunden, die im folgenden zusammenfassend als "erfindungsgemäße Verfahren" bezeichnet werden.

Des weiteren wurden die neuen thermisch oder thermisch und mit aktinischer Strahlung härtbaren Klebstoffe, Dichtungsmassen und Beschichtungsstoffe gefunden, die die erfindungsgemäßen Verbindungen und/oder die mit Hilfe der erfindungsgemäßen Verfahren hergestellten erfmdungsgemäßen Verbindungen enthalten und die im folgenden als "erfindungsgemäße Klebstoffe, Dichtungsmassen oder Beschichtungsstoffe" bezeichnet werden.

Darüber hinaus wurden die neuen Klebschichten, Dichtungen und Beschichtungen gefunden, die aus den erfindungsgemäßen Klebstoffen, Dichtungsmassen oder Beschichtungsstoffen herstellbar sind und im folgenden als "erfindungsgemäße Klebschichten, Dichtungen oder Beschichtungen" bezeichnet werden.

Weitere erfindungsgemäße Gegenstände gehen aus der folgenden Beschreibung hervor.

Im Hinblick auf den Stand der Technik war es für den Fachmann überraschend, daß die der vorliegenden Erfindung zugrundeliegende Aufgabe mit Hilfe der erfindungsgemäßen Verbindungen gelöst werden konnte. Hierbei überraschte insbesondere die leichte Zugänglichkeit der erfindungsgemäßen Verbindungen sowie ihre außerordentlich breite Verwendbarkeit.

Die erfindungsgemäßen Verbindungen enthalten primäre, sekundäre oder tertiäre Carbamatgruppen oder Allophanatgruppen. Im Hinblick auf die Verwendung in den erfindungsgemäßen Klebstoffen, Dichtungsmassen und Beschichtungsstoffen sind die primären und sekundären Carbamatgruppen und Allophanatgruppen von Vorteil und werden deshalb bevorzugt verwendet. Besondere Vorteile bieten indes primäre Carbamatgruppen und Allophanatgruppen, weswegen sie erfindungsgemäß besonders bevorzugt verwendet werden.

Die erfindungsgemäßen Verbindungen enthalten eine lineare C8-Kohlenstoffkette.

Bezüglich der beiden Ethylgruppen weist die lineare C8-Kohlenstoffkette das folgende Substitutionsmuste auf: 2,3, 2,4, 2,5, 2,6, 2,7, 3,4, 3,5, 3,6 oder 4,5. Erfindungsgemäß ist es von Vorteil, wenn die beiden Ethylgruppen in 2,4-Stellung stehen, d. h., daß es sich um 2,4-Diethyloctandioldicarbamate und 2,4-Diethyloctandioldiallophanate handelt.

Bezüglich der beiden Hydroxylgruppen weist die C8-Kohlenstoffkette das folgende Substitutionsmuster auf: 1,2, 1,3, 1,4, 1,5, 1,6, 1,7, 1,8, 2,3, 2,4, 2,5, 2,6, 2,7, 2,8, 3,4, 3,5, 3,6, 3,7, 3,8, 4,5, 4,6, 4,8, 5,6, 5,7, 5,8, 6,7, 6,8 oder 7,8. Erfindungsgemäß ist es von Vorteil, wenn die beiden Hydroxylgruppen in 1,5-Stellung stehen, d. h., daß es sich um Diethyloctan-1,5-dioldicarbamate oder um Diethyloctan-1,5-dioldiallophanate handelt.

Die beiden Substitutionsmuster werden in beliebiger Weise miteinander kombiniert, d. h., daß es sich bei den erfindungsgemäßen Verbindungen um
2,3-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol-,
2,4-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol-,
2,5-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol-,
2,6- Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol-,
2,7- Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol-,
3,4-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol-,
3,5-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol-,
3,6-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol- oder um
4,5-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-dioldicarbamate oder -diallophanate
handelt.

Von diesen erfindungsgemäßen Verbindungen weisen 2,4-Diethyloctan-1,5-dioldicarbamat und 2,4-Diethyloctan-1,5-dioldiallophanat, insbesondere aber 2,4-Diethyloctan-1,5-dioldicarbamat, bei der Herstellung und der Verwendung besondere Vorteile auf, weswegen sie erfindungsgemäß besonders bevorzugt verwendet werden.

Die Herstellung der erfindungsgemäßen Verbindungen geht aus von den stellungsisomeren Diethyloctandiolen.

Die erfindungsgemäß zu verwendenden stellungsisomeren Diethyloctandiol enthalten eine lineare C8-Kohlenstoffkette, deren Substitutionsmuster das Substitutionsmuster der erfindungsgemäßen Verbindungen festlegt.

Bezüglich der beiden Ethylgruppen weist die lineare C8-Kohlenstoffkette das folgende Substitutionsmuster auf: 2,3, 2,4, 2,5, 2,6, 2,7, 3,4, 3,5, 3,6 oder 4,5. Erfindungsgemäß ist es von Vorteil, wenn die beiden Ethylgruppen in 2,4-Stellung stehen, d. h., daß es sich um 2,4-Diethyloctandiole handelt.

Bezüglich der beiden Hydroxylgruppen weist die C8-Kohlenstoffkette das folgende Substitutionsmuster auf: 1,2, 1,3, 1,4, 1,5, 1,6, 1,7, 1,8, 2,3, 2,4, 2,5, 2,6, 2,7, 2,8, 3,4, 3,5, 3,6, 3,7, 3,8, 4,5, 4,6, 4,8, 5,6, 5,7, 5,8, 6,7, 6,8 oder 7,8. Erfindungsgemäß ist es von Vorteil, wenn die beiden Hydroxylgruppen in 1,5-Stellung stehen, d. h., daß es sich um Diethyloctan-1,5-diole handelt.

Die beiden Substitutionsmuster werden in beliebiger Weise miteinander kombiniert, d. h., daß es sich bei den erfindungsgemäß zu verwendenden Diethyloctandiolen um
2,3-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol,
2,4-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol,
2,5-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol,
2,6- Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol,
2,7- Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol,
3,4-Diethyloctan-1,2-, -1,3-, -1,4-, -1,5-, -1,6-, -1,7-, -1,8-, -2,3-; -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol,
3,5-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol,
3,6-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol oder um
4,5-Diethyloctan-1,2-, -1,3-, -1,4-, -1,5-, -1,6-, -1,7-, -1,8-, -2,3-, -2,4-, -2,5 -,-2,6-, -2,7-, -2,8-, -3,4-, -3,5-, -3,6-, -3,7-, -3,8 -, -4,5-, -4,6-, -4,7-, -4,8-, -5,6 -,-5,7-, -5,8-, -6,7-, -6,8- oder -7,8-diol
handelt.

Besondere Vorteile resultieren aus der Verwendung von 2,4-Diethyloctan-1,5-diol.

Die erfindungsgemäß zu verwendenden stellungsisomeren Diethyloctandiole sind an sich bekannte Verbindungen und können mit Hilfe üblicher und bekannter Synthesemethoden der organischen Chemie wie die basenkatalysierte Aldolkondensation hergestellt werden oder sie fallen als Nebenprodukte chemischer Großsynthesen wie der Herstellung von 2-Ethyl-hexanol an.

Die erfindungsgemäßen Verbindungen können in beliebiger geeigneter Weise nach den üblichen und bekannten Methoden der organischen Chemie, speziell der organischen Stickstoffchemie hergestellt werden. Erfindungsgemäß ist es jedoch von Vorteil, sie nach den erfindungsgemäßen Verfahren herzustellen.

Das erste erfindungsgemäße Verfahren zur Herstellung der erfindungsgemäßen stellungsisomeren Diethyloctandioldicarbamate umfaßt die Umsetzung der vorstehend beschriebenen stellungsisomeren Diethyloctandiole mit Alkyl-, Cycloalkyl- oder Arylcarbamaten, insbesondere, Methyl-, Butyl-, Cyclohexyl- oder Phenylcarbamat, wodurch die erfindungsgemäßen stellungsisomeren Diethyloctandioldicarbamate und Alkohole oder Phenole als Nebenprodukte resultieren. Die Nebenprodukte können in üblicher und bekannter Weise, beispielsweise durch Destillation, abgetrennt werden. Methodisch gesehen weist das erfindungsgemäß Verfahren keine Besonderheiten auf, sondern wird in Anlehnung an die in den Patentschriften US-A-4,758,632, US-A-4,301,257 oder US-A- 2,979,514 beschriebenen Bedingungen und Methoden durchgeführt.

Das zweite erfindungsgemäße Verfahren zu Herstellung der erfindungsgemäßen stellungsisomeren Diethyloctandioldicarbamate umfaßt die Umsetzung von stellungsisomeren Diethyloctandiolen mit Phosgen, wodurch die entsprechenden stellungsisomeren Chlorformiate resultieren.

Methodisch gesehen weist die Herstellung der Chlorformiatgruppen enthaltenden Zwischenprodukte keine Besonderheiten auf, sondern erfolgt nach den üblichen und bekannten Methoden der Phosgenchemie, wobei die entsprechenden geeigneten Anlagen verwendet und die für die Handhabung von Phosgen üblichen Sicherheitsmaßnahmen getroffen werden.

Vorteilhafterweise wird die Umsetzung mit Phosgen je nach Reaktivität der Diethyloctandiole und/oder der Rührbarkeit der betreffenden Lösung der Diethyloctandiole bei Temperaturen von -10 bis 100, bevorzugt 0 bis 50 und insbesondere 10 bis 40°C durchgeführt.

Zwar können die Chlorformiatgruppen enthaltenden Zwischenprodukte als solche isoliert werden, was für spezielle Fälle durchaus von Vorteil sein kann, im allgemeinen empfiehlt es sich aber die Zwischenprodukte in der Lösung, in der sie angefallen sind, mit Ammoniak und/oder primären und/oder sekundären Aminen umzusetzen.

Beispiele geeigneter primärer und sekundärer Amine sind solche der allgemeinen Formel I

**NHRR**^{**1**} (I),

worin die Variable R für ein Wasserstoffatom oder für einbindigen organischen Rest steht, der sich von den folgenden Verbindungen ableitet:
(i) Substituierte und unsubstiuierte, kein oder mindestens ein Heteroatom in der Kette und/oder im Ring enthaltende, lineare oder verzweigte Alkane, Alkene, Cycloalkane, Cycloalkene, Alkylcycloalkane, Alkylcycloalkene, Alkenylcycloalkane oder Alkenylcycloalkene;
(ii) substituierte und unsubstituierte Aromaten oder Heteroamaten; sowie
(iii) Alkyl-, Alkenyl-, Cycloalkyl-, Cycloalkenyl-, Alkylcyloalkyl-, Alkylcycloalkenyl-, Alkenylcycloalkyl- oder Alkenylcycloalkenylsubstitiuierte Aromaten oder Heteroaromaten, deren Substituenten substituiert oder unsubstituiert sind und kein oder mindestens ein Heteroatom in ihrer Kette und/oder ihrem Ring enthalten;
und worin die Variable R¹ die vorstehend angegebene Bedeutung hat, ausgenommen ein Wasserstoffatom;
oder worin die Reste R, ausgenommen ein Wasserstoffatom, und R¹ cyclisch miteinander verknüpft sind.

Beispiele geeigneter Heteroatome sind Sauerstoff-, Stickstoff-, Bor-, Silizium-, Schwefel- oder Phosphoratome.

Beispiele geeigneter Substitutienten, für die vorstehend genannten Reste R¹ sind Halogenatome, insbesondere Fluor- und Chloratome, Nitrogruppen oder Nitrilgruppen.

Beispiele geeigneter Aromaten sind Benzol und Naphthalin.

Beispiele geeigneter Heteroaromaten sind Thiophen, Pyridin oder Triazin.

Beispiele geeigneter Alkane sind solche mit 1 bis 20 C-Atomen im Molekül wie Methan Ethan, Propan, Butan, Isobutan, Pentan, Neopentan, Hexan, Heptan, Octan, Isooctan, Nonan, Dodecan, Hexadecan oder Eicosan.

Beispiele geeigneter Alkene sind Ethylen und Propylen.

Beispiele geeigneter Cycloalkane sind Cyclopentan und Cyclohexan.

Beispiele geeigneter Cycloalkene sind Cyclopenten und Cyclohexen.

Beispiele geeigneter Alkylcycloalkane sind Methylcyclopentan und Methylcyclohexan.

Beispiele geeigneter Alkylcycloalkene sind Methylcyclopenten und Methylcyclohexen.

Beispiele geeigneter Alkenylcycloalkane sind Allyl- und Vinylcyclopentan und Allyl- und Vinylcyclohexan.

Beispiele geeigneter Alkenylcycloalkene sind Vinylcyclopenten und Vinylcyclohexen.

Beispiele geeigneter Alkyl-, Alkenyl-, Cycloalkyl-, Cycloalkenyl-, Alkylcyloalkyl-, Alkylcycloalkenyl-, Alkenylcycloalkyl- oder Alkenylcycloalkenylsubstituenten sind Methyl, Ethyl, Propyl, Isopropyl, n-Butyl, sec.-Butyl, tert.-Butyl, Vinyl, Allyl, Cyclohexyl, Cyclohexenyl, 4-Methylcyclohexyl, 4-Methylcyclohexenyl, 3-Allylcyclohexenyl oder 4-Vinylcyclohexenyl.

Vorzugsweise leiten sich die Reste R' von organischen Verbindungen ab, die als solche unsubstituiert sind oder deren Substituenten unsubstituiert sind.

Vorteilhafterweise enthalten diese Verbindungen auch keine Heteroatome in ihren Ketten und/oder in ihren Ringen und/oder in den Ketten und/oder den Ringen ihrer Substitutienten.

Besondere Vorteile resultieren, wenn sich die Reste R und R¹ von linearen Alkanen ableiten, welche die vorstehend genannten vorteilhaften Bedingungen erfüllen. Weitere Vorteile resultieren, wenn sie sich von Methan, Ethan, Propan, Butan, Pentan oder Hexan ableiten.

Beispiele gut geeigneter primärer Amine 1 sind Methylamin, Ethylamin, Propylamin, Isobutylamin, Hexylamin, Cyclohexylamin, Allylamin, Cyclohexenylamin, Anilin, Cyclohexylmethylamin, (2-Cyclohexyl)ethylamin oder Benzylamin.

Durch die Umsetzung mit diesen primären Aminen I resultieren erfindungsgemäße Diethyloctandioldicarbamate mit sekundären Carbamatgruppen.

Beispiele gut geeigneter sekundärer Amine I sind Dimethylamin, Diethylamin, Methylethylamin, Dicyclohexylamin, Methylcyclohexylamin, Dibenzylamin, Methylbenzylamin oder Diphenylamin.

Beispiele gut geeigneter cyclischer Amine I sind Imidazol, Thiazin, Morpholin oder Piperidin.

Durch die Umsetzung mit diesen sekundären Aminen I resultieren erfindungsgemäße Diethyloctandioldicarbamate mit tertiären Carbamatgruppen.

Da erfindungsgemäße Diethyloctandioldicarbamate, die primäre Carbamatgruppen enthalten, ganz besonders vorteilhaft sind, wird Ammoniak erfindungsgemäß ganz besonders bevorzugt verwendet.

Methodisch gesehen weist die Umsetzung von Ammoniak und/oder von Aminen 1 mit den Chlorfomliatgruppen enthaltenden Zwischenprodukten keine Besonderheiten auf, sondern erfolgt nach den üblichen und bekannten Methoden der organischen Chemie. Die hierfür verwendeten Vorrichtungen und Verfahren richten sich vor allem danach, ob feste, flüssige oder gasförmige Amine I oder ob gasförmiges oder gelöstes Ammoniak eingesetzt wird oder werden. Der Fachmann kann daher in einfacher Weise die geeigneten Verfahren und Vorrichtungen aufgrund seines Fachwissens auswählen.

Die bei der Umsetzung der Chlorformiatgruppen mit Ammoniak oder den Aminen I anfallenden Ammoniumchloride werden in üblicher und bekannter Weise aus der das erfindungsgemäße Diethyloctandioldicarbamat enthaltenden Reaktionsmischung abgetrennt. Beispiele geeigneter Methoden sind die Filtration oder die Extraktion, wobei diese Methoden in geeigneter Weise miteinander kombiniert werden können.

Das erfindungsgemäße Verfahren zur Herstellung der erfindungsgemäßen stellungsisomeren Diethyloctandiolallophanate umfaßt die Umsetzung der stellungsisomeren Diethyloctandiole mit Alkyl-, Cycloalkyl- oder Arylallophanaten, insbesondere Alkylallophanaten, speziell Methyl- oder Ethylallophanat. Erfmdungsgemäß ist es von Vorteil, die Reaktion bei 50 bis 150 °C, vorzugsweise 60 bis 130 °C und insbesondere 80 bis 120 °C durchzuführen. Ein besonders guter Reaktionsverlauf wird gewährleistet, wenn man den Alkohol und/oder das Phenol laufend aus dem Reaktionsgemisch entfernt, beispielsweise durch Destillation im Vakuum. Um die Reaktion zu beschleunigen, kann dem Reaktionsgemisch noch ein üblich und bekannter saurer Katalysator wie p-Toluolsulfonsäure zugesetzt werden.

Je nach Verwendungszweck können die erfindungsgemäßen Verbindungen nach ihrer Herstellung und vor ihrer Verwendung isoliert werden, oder aber es können die Lösungen, in denen sie anfallen, direkt verwendet werden. Welcher Variante der Vorzug gegeben wird richtet sich vor allem nach dem Verwendungszweck. So wird man die erfindungsgemäßen Diethyloctandioldicarbamate bei ihrer Verwendung in lösemittelhahigen Klebstoffen, Dichtungsmassen und Beschichtungsstoffen in Lösung anwenden, wogegen sie vor ihrer Verwendung in lösemittelfreien, festen oder flüssigen Klebstoffen, Dichtungsmassen Beschichtungsstoffen isoliert werden.

Die erfindungsgemäßen Verbindungen können aufgrund ihrer neuartigen Strukturen als wertvolle Synthesebausteine in der niedermolekularen und hochmolekularen organischen Chemie und der metallorganischen Chemie verwendet werden.

Außerdem können die erfindungsgemäßen Verbindungen für die Herstellung thermisch und/oder mit aktinischer Strahlung härtbarer Klebstoffe, Dichtungsmassen und Beschichtungsstoffe verwendet werden. Insbesondere werden sie als Bestandteile der erfindungsgemäßen Klebstoffe, Dichtungsmassen und Beschichtungsstoffe angewandt. Hierbei kann ihr Anteil an den erfindungsgemäßen Klebstoffen, Dichtungsmassen und Beschichtungsstoffen außerordentlich breit variieren. Stellen die erfindungsgemäßen Verbindungen den Hauptbestandteil der erfindungsgemäßen Klebstoffe, Dichtungsmassen und Beschichtungsstoffe kann ihr Anteil hieran vorzugsweise bis zu 95, bevorzugt 90, besonders bevorzugt 85, ganz besonders bevorzugt 80 und insbesondere 75 Gew.-%, jeweils bezogen auf die erfindungsgemäßen Klebstoffe, Dichtungsmassen oder Beschichtungsstoffe, betragen. Die erfindungsgemäßen Verbindungen können indes auch als Zusatzstoffe in der Funktion von Reaktiverdünnern (vgl. hierzu Römpp Lexikon Lacke und Druckfarben, Georg Thieme Verlag, Stuttgart, New York, 1998, »Reaktivverdünner«, S. 491) für die thermische Vernetzung angewandt werden. Hierbei genügt bereits ein Anteil von vorzugsweise 0,1 bis 20, bevorzugt 0,2 bis 18, besonders bevorzugt 0,3 bis 16, ganz besonders bevorzugt 0,4 bis 14 und insbesondere 0,5 bis 12 Gew.-%, jeweils bezogen auf die erfmdungsgemäßen Klebstoffe, Dichtungsmassen oder Beschichtungsstoffe, um die erfindungsgemäßen Vorteile zu erzielen.

Die erfindungsgemäßen Klebstoffe, Dichtungsmassen oder Beschichtungsstoffe können darüber hinaus übliche und bekannte Bindemittel, Vernetzungsmittel und Zusatzstoffe in wirksamen Mengen enthalten.

Die Bindemittel können den unterschiedlichsten Oligomer- und Polymerklassen entstammen. Beispiele geeigneter Oligomer- und Polymerklassen sind statistisch, alternierend und/oder blockartig aufgebaute lineare und/oder verzweigte und/oder kammartig aufgebaute (Co)Polymerisate von ethylenisch ungesättigten Monomeren, oder Polyadditionsharze und/oder Polykondensationsharze. Zu diesen Begriffen wird ergänzend auf Römpp Lexikon Lacke und Druckfarben, Georg Thieme Verlag, Stuttgart, New York, 1998, Seite 457, »Polyaddition« und »Polyadditionsharze (Polyaddukte)«, sowie Seiten 463 und 464, »Polykondensate«, »Polykondensation« und »Polykondensationsharze« verwiesen. Hinsichtlich möglicherweise vorhandener Substituenten gilt das vorstehend Gesagte sinngemäß.

Beispiele gut geeigneter (Co)Polymerisate sind Poly(meth)acrylate und partiell verseifte Polyvinylester.

Beispiele gut geeigneter Polyadditionsharze und/oder Polykondensationsharze sind Polyester, Alkyde, Polyurethane, Polylactone, Polycarbonate, Polyether, Epoxidharz-Amin-Addukte, Polyharnstoffe, Polyamide oder Polyimide.

Besondere Vorteile resultieren, wenn die vorstehend beschriebenen Bindemittel carbamatreaktive funktionelle Gruppen wie N-Methylol- oder N-Methylolethergruppen enthalten.

Beispiele geeigneter Vernetzungsmittel sind Aminoplastharze. Beispiele geeigneter Aminoplastharze sind üblich und bekannt, und zahlreiche Produkte sind im Handel erhältlich.

Beispiele gut geeigneter Aminoplastharze sind Melaminharze, Guanaminharze oder Harnstoffharze. Hierbei kann jedes für transparente Decklacke oder Klarlacke geeignete Aminoplastharz oder eine Mischung aus solchen Aminoplastharzen verwendet werden. Ergänzend wird auf Römpp Lexikon Lacke und Druckfarben, Georg Thieme Verlag, 1998, Seite 29, »Aminoharze«, und das Lehrbuch "Lackadditive" von Johan Bieleman, Wiley-VCH, Weinheim, New York, 1998, Seiten 242 ff., oder auf das Buch "Paints, Coatings and Solvents", second completely revised edition, Edit. D. Stoye und W. Freitag, Wiley-VCH, Weinheim, New York, 1998, Seiten 80 ff., verwiesen. Des weiteren kommen die üblichen und bekannten Aminoplastharze in Betracht, deren Methylol- und/oder Methoxymethylgruppen z. T. mittels Carbamat- oder Allophanatgruppen defunktionalisiert sind. Vernetzungsmittel dieser Art werden in den Patentschriften US-A-4 710 542 und EP-B-0 245 700 sowie in dem Artikel von B. Singh und Mitarbeiter "Carbamylmethylated Melamines, Novel Crosslinkers for the Coatings Industry" in Advanced Organic Coatings Science and Technology Series, 1991, Band 13, Seiten 193 bis 207, beschrieben.

Außer diesen Vemetzungsmitteln können noch weitere Vemetzungsmittel vorhanden sein. Beispiele geeigneter weiterer Vernetzungsmittel sind Siloxangruppen enthaltende Verbindungen oder Harze, Anhydridgruppen enthaltende Verbindungen oder Harze, Epoxidgruppen enthaltende Verbindungen oder Harze, blockierte und/oder unblockierte Polyisocyanate und/oder Tris(alkoxycarbonylamino)triazine wie sie in den Patentschriften US-A-4 939 213.US-A-5 084 541, US-A-5 288 865 oder EP-A-0 604 922 beschrieben werden.

Je nach der Reaktivität des weiteren Vernetzungsmittels kann es den erfindungsgemäßen Beschichtungsstoffen, Klebstoffen und Dichtungsmassen direkt zugesetzt werden, wodurch ein sogenanntes Einkomponentensystem resultiert. Handelt es sich indes um ein besonders reaktives Vernetzungsmittel, wie ein Polyisocyanat oder ein Epoxid, wird dieses im allgemeinen erst kurz vor der Verwendung den erfindungsgemäßen Beschichtungsstoffen, Klebstoffen und Dichtungsmassen zugesetzt. Hierbei resultiert ein sogenanntes Zwei- oder Mehrkomponentensystem.

Sollen die erfindungsgemäßen Beschichtungsstoffe, Klebstoffe und Dichtungsmassen nicht nur thermisch sondern auch mit aktinischer Strahlung härtbar sein, enthalten sie übliche und bekannte Bestandteile, die mit aktinischer Strahlung aktivierbar sind. Im Rahmen der vorliegenden Erfindung ist unter aktinischer Strahlung elektromagnetische Strahlung, insbesondere sichtbares Licht, UV-Licht oder Röntgenstrahlung, oder Korpuskularstrahlung, insbesondere Elektronenstrahlung, zu verstehen. Besonders bevorzugt wird UV-Licht angewandt. Beispiele geeigneter Bestandteile, die mit aktinischer Strahlung aktivierbar sind, sind (meth)acryl-, allyl-, vinyl- oder dicyclopentadienylfunktionelle (Meth)Acrylatcopolymere oder Polyetheracrylate, Polyesteracrylate, ungesättigte Polyesteracrylate, Epoxyacrylate, Urethanacrylate, Aminoacrylate, Melaminacrylate, Silikonacrylate oder die entsprechenden Methacrylate.

Beispiele geeigneter Zusatzstoffe sind Katalysatoren für die Vernetzung, Initiatoren, insbesondere Photoinitiatoren, Pigmente, Farbstoffe, Füllstoffe, Verstärkerfüllstoffe, Rheologiehilfsmittel, Lösemittel, Netz- und Dispergiermittel, Entschäumer, Haftvermittler, Additive zur Verbesserung der Untergrundbenetzung, Additive zur Verbesserung der Oberflächenglätte, Mattierungsmittel, Verlaufmittel, Filmbildehilfsmittel, Trockenstoffe, Hautverhinderungsmittel, Lichtschutzmittel, Korrosionsinhibitoren, Biozide, Flammschutzmittel, Polymerisationsinhibitoren, insbesondere Photoinhibitoren, oder Weichmacher, wie sie beispielsweise auf dem Kunststoff- oder Lacksektor üblich und bekannt sind. Weitere Beispiele geeigneter Zusatzstoffe (C) werden in dem Lehrbuch »Lackadditive« von Johan Bieleman, Wiley-VCH, Weinheim, New York, 1998, beschrieben.

Die Auswahl der Zusatzstoffe richtet sich nach dem gewünschten Eigenschaftsprofil der erfindungsgemäßen Beschichtungsmittel, Klebstoffe und Dichtungsmassen sowie deren speziellen Verwendungszwecken und kann daher vom Fachmann in einfacher Weise, gegebenenfalls unter Zuhilfenahme einfacher Vorversuche, getroffen werden.

Die erfindungsgemäßen Klebstoffe, Dichtungsmassen und Beschichtungsstoffe können in wäßrigen, wäßrig organischen oder organischen Medien gelöst oder dispergiert oder als sogenannte "NAD" (non-aqueous dispersion) vorliegen. Außerdem können sie fein verteilt in fester Form, beispielsweise als Pulverlacke, oder in fester Form in Wasser dispergiert, beispielsweise als Pulverslurries, vorliegen. Des weiteren können sie in lösemittelfreier flüssiger Form als sogenannte 100%-Systeme vorliegen. Die jeweils erforderlichen Bestandteile der erfindungsgemäßen Klebstoffe, Dichtungsmassen und Beschichtungsstoffe kann der Fachmann leicht anhand des vorgegebenen Eigenschaftsprofils (fest, flüssig, in organischen Lösemittel löslich, wasserlöslich usw.) auswählen.

Die Herstellung der erfindungsgemäßen Klebstoffe, Dichtungsmassen und Beschichtungsstoffe weist keine Besonderheiten auf, sondern erfolgt in üblicher und bekannter Weise durch Vermischen der vorstehend beschriebenen Bestandteile in geeigneten Mischaggregaten wie Rührkessel, Dissolver, Rührwerksmühlen oder Extruder nach den für die Herstellung der jeweiligen erfindungsgemäßen Klebstoffe, Dichtungsmassen und Beschichtungsstoffe geeigneten Verfahren.

Die erfindungsgemäßen Klebstoffe dienen der Herstellung erfindungsgemäßer Klebschichten auf grundierten und ungrundierten Substraten.

Die erfindungsgemäßen Dichtungsmassen dienen der Herstellung erfindungsgemäßer Dichtungen auf und in grundierten und ungrundierten Substraten.

Die erfindungsgemäßen Beschichtungsstoffe können als Füller, Unidecklacke, Basislacke und Klarlacken verwendet werden und dienen der Herstellung ein- oder mehrschichtiger Klarlackierungen oder farb- und/oder effektgebender Lackierungen auf grundierten und ungrundierten Substraten.

Ganz besondere Vorteile resultieren bei ihrer Verwendung zur Herstellung von Klarlackierungen, insbesondere im Rahmen des sogenannten Naß-in-naß-Verfahrens, bei dem ein Basislack, insbesondere ein Wasserbasislack, auf das grundierte oder ungrundierte Substrat appliziert und getrocknet, indes nicht gehärtet wird, wonach man auf die Basislackschicht einen Klarlack appliziert und die resultierende Klarlackschicht gemeinsam mit der Basislackschicht thermisch oder thermisch und mit aktinischer Strahlung härtet.

Als Substrate kommen alle zu lackierenden Oberflächen, die durch eine Härtung der hierauf befindlichen Schichten unter der Anwendung von Hitze oder der kombinierten Anwendung von Hitze und aktinischer Strahlung nicht geschädigt werden, in Betracht; das sind z. B. Metalle, Kunststoffe, Holz, Keramik, Stein, Textil, Faserverbunde, Leder, Glas, Glasfasern, Glas- und Steinwolle, mineral- und harzgebundene Baustoffe, wie Gips- und Zementplatten oder Dachziegel, sowie Verbunde dieser Materialien. Demnach sind die erfindungsgemäßen Lackierung, Klebschichten oder Dichtung auch für Anwendungen außerhalb der Automobilerstlackierung und der Autoreparaturlackierung geeignet. Hierbei kommen sie insbesondere für die Lackierung, das Verkleben und/oder das Abdichten von Möbeln und für die industrielle Anwendung, inklusive Coil Coating, Container Coating und die Imprägnierung oder Beschichtung elektrotechnischer Bauteile, in Betracht. Im Rahmen der industriellen Anwendungen eignen sie sich für die Lackierung, das Verkleben und/oder das Abdichten praktisch aller Teile für den privaten oder industriellen Gebrauch wie Radiatoren, Haushaltsgeräte, Kleinteile aus Metall wie Schrauben und Muttern, Radkappen, Felgen, Emballagen oder elektrotechnische Bauteile wie Motorwicklungen oder Transformatorwicklungen.

Im Falle elektrisch leitfähiger Substrate können Grundierungen verwendet werden, die in üblicher und bekannter Weise aus Elektrotauchlacken (ETL) hergestellt werden. Hierfür kommen sowohl anodische (ATL) als auch kathodische (KTL) Elektrotauchlacke, insbesondere aber KTL, in Betracht.

Es können auch grundierte oder ungrundierte Kunststoffteile aus z. B. ABS, AMMA, ASA, CA, CAB, EP, UF, CF, MF, MPF, PF, PAN, PA, PE, HDPE, LDPE, LLDPE, UHMWPE, PC, PC/PBT, PC/PA, PET, PMMA, PP, PS, SB, PUR, PVC, RF, SAN, PBT, PPE, POM, PUR-RIM, SMC, BMC, PP-EPDM und UP (Kurzbezeichnungen nach DIN 7728T1) lackiert, geklebt oder abgedichtet werden. Im Falle von nichtfunktionalisierten und/oder unpolaren Substratoberflächen können diese vor der Beschichtung in bekannter Weise einer Vorbehandlung, wie mit einem Plasma oder mit Beflammen, unterzogen oder mit einer Hydrogrundierung versehen werden.

Die Applikation der erfindungsgemäßen Klebstoffe, Dichtungsmassen und Beschichtungsstoffe kann durch alle üblichen Applikationsmethoden, wie z.B. Spritzen, Rakeln, Streichen, Gießen, Tauchen, Tränken, Träufeln oder Walzen erfolgen. Dabei kann das zu beschichtende Substrat als solches ruhen, wobei die Applikationseinrichtung oder -anlage bewegt wird. Indes kann auch das zu beschichtende Substrat, insbesondere ein Coil, bewegt werden, wobei die Applikationsanlage relativ zum Substrat ruht oder in geeigneter Weise bewegt wird. Enthalten die erfindungsgemäßen Klebstoffe, Dichtungsmassen und Beschichtungsstoffe Bestandteile, die mit aktinischer Strahlung aktivierbar sind, wird die Applikation vorzugsweise unter Ausschluß von Licht durchgeführt.

Die applizierten Schichten aus den erfindungsgemäßen Klebstoffen, Dichtungsmassen und Beschichtungsstoffen werden in üblicher und bekannter Weise - gegebenenfalls nach einer gewissen Ruhezeit, die dem Verlauf der Schichten und/oder dem Verdampfen flüchtiger Bestandteile dient - thermisch oder thermisch und mit aktinischer Strahlung gehärtet.

Methodisch weist die thermische Härtung keine Besonderheiten auf, sondern es werden die üblichen und bekannten Temperaturen im Bereich von Raumtemperatur bis 200 °C, Härtungszeiten im Bereich von einer Minute bis drei Stunden und Vorrichtungen wie Heizstrahler oder Umluftöfen angewandt.

Auch die Härtung mit aktinischer Strahlung weist keine methodischen Besonderheiten auf, sondern erfolgt in üblicher und bekannter Weise durch Bestrahlen mit UV-Lampen und/oder Elektronenstrahlquellen vorzugsweise unter Inertgas.

Bei der Härtung der erfindungsgemäßen Dual Cure-Klebstoffe, -Dichtungsmassen und -Beschichtungsstoffe können die thermische Härtung und Härtung mit aktinischer Strahlung gleichzeitig oder alternierend eingesetzt werden. Werden die beiden Härtungsmethoden alternierend verwendet, kann beispielsweise mit der thermischen Härtung begonnen und mit der Härtung mit aktinischer Strahlung geendet werden. In anderen Fällen kann es sich als vorteilhaft erweisen, mit der Härtung mit aktinischer Strahlung zu beginnen und hiermit zu enden. Der Fachmann kann die Härtungsmethode, welche für den jeweiligen Einzelfall am vorteilhaftesten ist, aufgrund seines allgemeinen Fachwissens gegebenenfalls unter Zuhilfenahme einfacher Vorversuche ermitteln.

Die aus den erfmdungsgemäßen und Dichtungsmassen hergestellten erfindungsgemäßen Klebschichten und Dichtungen haben auch unter extremen klimatischen Bedingungen eine hervorragende Klebkraft und Dichtungsfähigkeit auch über lange Zeiträume hinweg.

Die aus den erfindungsgemäßen Beschichtungsstoffen hergestellten erfindungsgemäßen Beschichtungen weisen einen hervorragenden Verlauf und einen hervorragenden optischen Gesamteindruck auf. Sie sind witterungsstabil, säurebeständig und feuchtigkeitsbeständig und vergilben auch im tropischen Klima nicht. Sie sind daher im Innen- und Außenbereich verwendbar.

Daher zeichnen sich auch die erfindungsgemäßen grundierten und ungrundierten Substrate, insbesondere Karosserien von Automobilen und Nutzfahrzeugen, industrielle Bauteile, inklusive Kunstsstoffteile, Emballagen, Coils und elektrische Bauteile, oder Möbel, die mit mindestens einer erfindungsgemäßen Beschichtung beschichtet, mit mindestens einer erfindungsgemäßen Dichtung abgedichtet und/oder mit mindestens einem erfindungsgemäßen Klebstoff verklebt sind, durch besondere technische und wirtschaftliche Vorteile, insbesondere eine lange Gebrauchsdauer, aus, was sie für die Anwender besonders attraktiv macht.

### Beispiele

### Beispiel 1

### Herstellung von 2,4-Diethyloctan-1,5-dioldicarbamat

2,4-Diethyloctan-1,5-diol wurden in Xylol homogen gelöst. Die resultierende Lösung wurde in einem geeigneten Reaktor vorgelegt. Anschließend wurden unter Rühren bei Raumtemperatur während einer Stunde, bezogen auf die vorhandenen Hydroxylgruppen, ein molarer Überschuß Phosgen zur Lösung zudosiert. Nach 30minütiger Nachreaktionszeit wurde der Grad der Umsetzung IR-spektroskopisch ermittelt. Hierbei waren keine Hydroxylgruppen mehr nachweisbar. Das überschüssige Phosgen wurde unter Vakuum abgetrennt. Anhand der Chlorwertbestimmung wurde ein Umsatz >90% festgestellt.

Die Lösung des Zwischenpodukts wurden in einem geeigneten Reaktor vorgelegt. Anschließend wurden bei Raumtemperatur, bezogen auf die vorhandenen Chlorformiatgruppen, gasförmiges Ammoniak in die Lösung eingeleitet, wobei Ammoniumchlorid ausfiel.

Die resultierende Lösung wurde nacheinander mit Wasser, Ethanol und Pentylacetat versetzt, um das ausgefallene Ammoniumchlorid zu lösen und eine Phasentrennung hervorzurufen. Die resultierende Mischung wurde während einer Stunde bei Raumtemperatur gerührt und dann zur Phasentrennung stehen gelassen. Nach erfolgter Phasentrennung wurde die wäßrige Phase abgetrennt, und die organische Phase wurde mit Natriumchlorid getrocknet.

Die NMR-Analyse ergab, daß die Chlorformiatgruppen zu mehr als 80% zu Carbamatgruppen umgesetzt waren. Das 2,4-Diethyloctan-1,5-dioldicarbamat wurde durch Eindampfen der Lösung isoliert.

### Herstellbeispiel 1

### Der Herstellung eines Bindemittels zur Verwendung in einem erfindungsgemäßen Klarlack

In einem geeigneten Reaktionsgefäß, ausgerüstet mit Rührer, Rückflußkühler und zwei Zulaufgefäßen, wurden 100 Gewichtsteile einer Mischung aus 2,4-Diethylactan-1,5-diol, Methylamylketon und Ethoxyethylpropionat (Gewichtsverhältnis: 1:1:1) vorgelegt und auf 145 °C aufgeheizt. Zu der Vorlage dosierte man unter Rühren gleichmäßig innerhalb von 4,5 Stunden eine Mischung aus 100 Gewichtsteilen VeoVa® 10 (vgl. Römpp Lexikon Lacke und Druckfarben, Georg Thieme Verlag, Stuttgart, New York, 1998, »VeoVa®«, Seite 598), 150 Gewichtsteil Styrol, 100 Gewichtsteile tert.-Butylcyclohexylacrylat, 200 Gewichtsteile n-Butylmethacrylat, 10 Gewichtsteile 2-Hydroxyethylacrylat und 100 Gewichtsteile Isodecylmethacrylat. 15 Minuten vor Beginn dieses Zulaufs wurde mit dem Zulauf einer Mischung aus 40 Gewichtsteilen Di-tert.-butylperoxid und Methylamylketon begonnen. Diese Mischung wurde während fünf Stunden gleichmäßig zu der resultierenden Reaktionsmischung zudosiert. Nach der Nachpolymerisation wurde die Reaktionsmischung mit Methoxypropylacetat auf einen Feststoffgehalt von 74 Gew.-% (eine Stunde bei 130°C) eingestellt.

### Beispiel

### Herstellung und Applikation eines erfindungsgemäßen Klarlacks

1 Gewichtsteil 2,4-Diethyloctan-1,5-dioldicarbamat (vgl. Beispiel 1), 1 Gewichtsteil eines handelsüblichen Melaminharzes (Luwipal® 066 der Firma BASF Aktiengesellschaft), 121,62 Gewichtsteile der Bindemittellösung gemäß dem Herstellbeispiel 1 und 0,28 Gewichtsteile eines handelsüblichen sauren Vemetzungskatalysators (Nacure® 4575) wurden miteinander vermischt. Der resultierende Klarlack wurde auf Glas in einer Naßschichtdicke von 100 µm appliziert und während 30 Minuten bei 130 °C gehärtet. Die resultierende erfindungsgemäße Klarlackierung war klar, transparent, kratzfest und säurebeständig.

## Patentansprüche

1. Stellungsisomere Diethyloctandioldicarbamate und Diethyloctandioldiallophanate.

2. Die stellungsisomeren Diethyloctandioldicarbamate und Diethyloctandioldiallophanate nach Anspruch 1, **dadurch gekennzeichnet, daß** sie primäre Carbamat- oder Allophanatgruppen enthalten.

3. Die stellungsisomeren Diethyloctandioldicarbamate und Diethyloctandioldiallophanate nach Anspruch 1 oder 2, **dadurch gekennzeichnet, daß** ihre lineare C8-Kohlenstoffkette bezüglich der beiden Ethylgruppen das folgende Substitutionsmuster aufweisen: 2,3, 2,4, 2,5, 2,6, 2,7, 3,4, 3,5, 3,6 oder 4,5.

4. Die stellungsisomeren Diethyloctandioldicarbamate und Diethyloctandioldiallophanate nach Anspruch 3, **dadurch gekennzeichnet, daß** die beiden Ethylgruppen in 2,4-Stellung stehen.

5. Die stellungsisomeren Diethyloctandioldicarbamate und Diethyloctandioldiallophanate nach einem der Ansprüche 1 bis 4, **dadurch gekennzeichnet, daß** ihre C8-Kohlenstoffkette bezüglich der beiden Carbamatgruppen oder Allophanatgruppen das folgende Substitutionsmuster aufweisen: 1,2, 1,3, 1,4, 1,5, 1,6, 1,7, 1,8, 2,3, 2,4, 2,5, 2,6, 2,7, 2,8, 3,4, 3,5, 3,6, 3,7, 3,8, 4,5, 4,6, 4,8, 5,6, 5,7, 5,8, 6,7, 6,8 oder 7,8.

6. Die stellungsisomeren Diethyloctandioldicarbamate und Diethyloctandioldiallophanate nach Anspruch 5, **dadurch gekennzeichnet, daß** die beiden Carbamatgruppen oder Allophanatgruppen in 1,5-Stellung stehen.

7. Die stellungsisomeren Diethyloctandioldicarbamate und Diethyloctandioldiallophanate nach Anspruch 6, **dadurch gekennzeichnet, daß** es sich um 2,4-Diethyloctan-1,5-dioldicarbamat und um 2,4-Diethyloctan-1,5-dioldiallophanat handelt.

8. Verfahren zur Herstellung von stellungsisomeren Diethyloctandioldicarbamaten gemäß einem der Ansprüche 1 bis 7 aus Diethyloctandiolen, **dadurch gekennzeichnet, daß** es die Umsetzung von stellungsisomeren Diethyloctandiolen mit Alkyl-, Cycloalkyl- oder Arylcarbamaten umfaßt.

9. Verfahren zur Herstellung von stellungsisomeren Diethyloctandioldicarbamaten gemäß einem der Ansprüche 1 bis 7aus Diethyloctandiolen, **dadurch gekennzeichnet, daß** es die Umsetzung von stellungsisomeren Diethyloctandiolen mit Phosgen zu den entsprechenden Chlorformiaten und Umsetzung dieser Zwischenprodukte mit Ammoniak und/oder Aminen umfaßt.

10. Verfahren zur Herstellung von stellungsisomeren Diethyloctandioldiallophanaten gemäß einem der Ansprüche 1 bis 7 aus Diethyloctandiolen, **dadurch gekennzeichnet, daß** es die Umsetzung von stellungsisomeren Diethyloctandiolen mit Alkyl-, Cycloalkyl- oder Arylallophanaten umfaßt.

11. Das Verfahren nach einem der Ansprüche 8 bis 10, **dadurch gekennzeichnet, daß** die lineare C8-Kohlenstoffkette der stellungsisomeren Diethyloctandiole bezüglich der beiden Ethylgruppen das folgende Substitutionsmuster aufweisen: 2,3, 2,4, 2,5, 2,6, 2,7, 3,4, 3,5, 3,6 oder 4,5.

12. Das Verfahren nach Anspruch 11, **dadurch gekennzeichnet, daß** die beiden Ethylgruppen in 2,4-Stellung stehen.

13. Das Verfahren nach einem der Ansprüche 8 bis 12, **dadurch gekennzeichnet, daß** die C8-Kohlenstoffkette der stellungsisomeren Diethyloctandiole bezüglich der beiden Hydroxylgruppen das folgende Substitutionsmuster aufweisen: 1,2, 1,3, 1,4, 1,5, 1,6, 1,7, 1,8, 2,3, 2,4, 2,5, 2,6, 2,7, 2,8, 3,4, 3,5, 3,6, 3,7, 3,8, 4,5, 4,6, 4,8, 5,6, 5,7, 5,8, 6,7, 6,8 oder 7,8.

14. Das Verfahren nach Anspruch 13, **dadurch gekennzeichnet, daß** die beiden Hydroxylgruppen in 1,5-Stellung stehen.

15. Das Verfahren nach Anspruch 14, **dadurch gekennzeichnet, daß** man 2,4-Diethyloctan-1,5-diol verwendet.

16. Verwendung der stellungsisomeren Diethyloctandioldicarbamate und Diethyloctandioldiallophanate gemäß einem der Ansprüche 1 bis 7, der mit Hilfe des Verfahrens gemäß einem der Ansprüche 8, 9 oder 11 bis 15 hergestellten stellungsisomeren Diethyloctandioldicarbamate und der mit Hilfe des Verfahrens gemäß einem der Ansprüche 10 bis 15 hergestellten Diethyloctandioldiallophanate als Synthesebausteine in der niedermolekularen und hochmolekularen organischen Chemie und der metallorganischen Chemie.

17. Verwendung der stellungsisomeren Diethyloctandioldicarbamate und Diethyloctandioldiallophanate gemäß einem der Ansprüche 1 bis 7, der mit Hilfe des Verfahrens gemäß einem der Ansprüche 8, 9 oder 11 bis 15 hergestellten stellungsisomeren Diethyloctandioldicarbamate und der mit Hilfe des Verfahrens gemäß einem der Ansprüche 10 bis 15 hergestellten Diethyloctandioldiallophanate für die Herstellung thermisch oder thermisch und mit aktinischer Strahlung härtbarer Klebstoffe, Dichtungsmassen und Beschichtungsstoffe.

18. Thermisch oder thermisch und mit aktinischer Strahlung härtbare Klebstoffe, Dichtungsmassen und Beschichtungsstoffe, **dadurch gekennzeichnet, daß** sie mindestens eines der Diethyloctandioldicarbamate und/oder mindestens eines der Diethyloctandioldiallophanate gemäß einem der Ansprüche 1 bis 7 und/oder mindestens eines der mit Hilfe des Verfahrens gemäß einem der Ansprüche 8, 9 oder 11 bis 15 hergestellten Diethyloctandioldicarbamate und/oder mindestens eines der mit Hilfe des Verfahrens gemäß einem der Ansprüche 11 bis 15 hergestellten Diethyloctandioldiallophanate enthalten.

19. Klebschichten, Dichtungen und Beschichtungen auf und in grundierten und ungrundierten Substraten, herstellbar aus den thermisch oder thermisch und mit aktinischer Strahlung härtbaren Klebstoffen, Dichtungsmassen und Beschichtungsstoffe gemäß Anspruch 18.

20. Grundierte und ungrundierte Substrate mit Klebschichten, Dichtungen und/oder Beschichtungen gemäß Anspruch 19.

## Claims

1. Positionally isomeric diethyloctanediol dicarbamates and diethyloctanediol diallophanates.

2. The positionally isomeric diethyloctanediol dicarbamates and diethyloctanediol diallophanates as claimed in claim 1, which contain primary carbamate or allophanate groups.

3. The positionally isomeric diethyloctanediol dicarbamates and diethyloctanediol diallophanates as claimed in claim 1 or 2, whose linear C8 carbon chain has the following substitution pattern with regard to the two ethyl groups: 2,3, 2,4, 2,5, 2,6, 2,7, 3,4, 3,5, 3,6 or 4,5.

4. The positionally isomeric diethyloctanediol dicarbamates and diethyloctanediol diallophanates as claimed in claim 3, wherein the two ethyl groups are in positions 2 and 4.

5. The positionally isomeric diethyloctanediol dicarbamates and diethyloctanediol diallophanates as claimed in any of claims 1 to 4, whose C8 carbon chain has the following substitution pattern with regard to the two carbamate groups or allophanate groups: 1,2, 1,3, 1,4, 1,5, 1,6, 1,7, 1,8, 2,3, 2,4, 2,5, 2,6, 2,7, 2,8, 3,4, 3,5, 3,6, 3, 7, 3,8, 4, 5, 4, 6, 4, 8, 5,6, 5,7, 5, 8, 6, 7, 6,8 or 7,8.

6. The positionally isomeric diethyloctanediol dicarbamates and diethyloctanediol diallophanates as claimed in claim 5, wherein the two carbamate groups or allophanate groups are in positions 1 and 5.

7. The positionally isomeric diethyloctanediol dicarbamates and diethyloctanediol diallophanates as claimed in claim 6, which are 2,4-diethyloctane-1,5-diol dicarbamate and 2,4-diethyloctane-1,5-diol diallophanate.

8. A process for preparing positionally isomeric diethyloctanediol dicarbamates as claimed in any of claims 1 to 7 from diethyloctanediols, which comprises reacting positionally isomeric diethyloctanediols with alkyl, cycloalkyl or aryl carbamates.

9. A process for preparing positionally isomeric diethyloctanediol dicarbamates as claimed in any of claims 1 to 7 from diethyloctanediols, which comprises the reaction of positionally isomeric diethyloctanediols with phosgene to give the corresponding chloroformates and reacting these intermediates with ammonia and/or amines.

10. A process for preparing positionally isomeric diethyloctanediol diallophanates as claimed in any of claims 1 to 7 from diethyloctanediols, which comprises the reaction of positionally isomeric diethyloctanediols with alkyl, cycloalkyl or aryl allophanates.

11. The process as claimed in any of claims 8 to 10, wherein the linear C8 carbon chain of the positionally isomeric diethyloctanediols has the following substitution pattern with regard to the two ethyl groups: 2,3, 2,4, 2,5, 2,6, 2,7, 3,4, 3 , 5, 3, 6 or 4,5.

12. The process as claimed in claim 11, wherein the two ethyl groups are in positions 2 and 4.

13. The process as claimed in any of claims 8 to 12, wherein the C8 carbon chain of the positionally isomeric diethyloctanediols has the following substitution pattern with regard to the two hydroxyl groups: 1,2, 1,3, 1,4, 1,5, 1,6, 1,7, 1,8, 2,3, 2,4, 2,5, 2,6, 2,7, 2,8, 3,4, 3,5, 3,6, 3,7, 3,8, 4,5, 4,6, 4,8, 5,6, 5,7, 5,8, 6,7, 6,8 or 7,8.

14. The process as claimed in claim 13, wherein the two hydroxyl groups are in positions 1 and 5.

15. The process as claimed in claim 14, wherein 2,4-diethyloctane-1,5-diol is used.

16. The use of the positionally isomeric diethyloctanediol dicarbamates and diethyloctanediol diallophanates as claimed in any of claims 1 to 7, of the positionally isomeric diethyloctanediol dicarbamates prepared with the aid of the process as claimed in any of claims 8, 9 and 11 to 15, and of the diethyloctanediol diallophanates prepared with the aid of the process as claimed in any of claims 10 to 15 as synthesis building blocks in low-molecular and high-molecular organic chemistry and in organometallic chemistry.

17. The use of the positionally isomeric diethyloctanediol dicarbamates and diethyloctanediol diallophanates as claimed in any of claims 1 to 7, of the positionally isomeric diethyloctanediol dicarbamates prepared with the aid of the process as claimed in any of claims 8, 9 and 11 to 15, and of the diethyloctanediol diallophanates prepared with the aid of the process as claimed in any of claims 10 to 15 for the preparation of adhesives, sealing compounds, and coating materials curable thermally, or thermally and with actinic radiation.

18. Adhesives, sealing compounds and coating materials curable thermally, or thermally and with actinic radiation, comprising at least one of the diethyloctanediol dicarbamates and/or at least one of the diethyloctanediol diallophanates as claimed in any of claims 1 to 7 and/or at least one of the diethyloctanediol dicarbamates prepared with the aid of the process as claimed in any of claims 8, 9 and 11 to 15 and/or at least one of the diethyloctanediol diallophanates prepared with the aid of the process as claimed in any of claims 10 to 15.

19. Adhesive films, seals, and coatings on and in primed and unprimed substrates, which can be produced from the adhesives, sealing compounds, and coating materials, as claimed in claim 18, curable thermally, or thermally and with actinic radiation.

20. Primed and unprimed substrates with adhesive films, seals and/or coatings as claimed in claim 19.

## Revendications

1. Isomères de position de dicarbamates de diéthyloctanediol et de diallophanates de diéthyloctanediol.

2. Isomères de position de dicarbamates de diéthyloctanediol et de diallophanates de diéthyloctanediol selon la revendication 1, **caractérisés en ce qu'**ils contiennent des groupes primaires de carbamate ou d'allophanate.

3. Isomères de position de dicarbamates de diéthyloctanediol et de diallophanates de diéthyloctanediol selon la revendication 1 ou 2, **caractérisés en ce que** leurs chaînes linéaires de carbone en C8 présentent par rapport aux deux groupes éthyle le motif de substitution suivant: 2,3, 2,4, 2,5, 2,6, 2,7, 3,4, 3,5, 3,6 ou 4,5.

4. Isomères de position de dicarbamates de diéthyloctanediol et de diallophanates de diéthyloctanediol selon la revendication 3, **caractérisés en ce que** les deux groupes éthyle sont en position 2,4.

5. Isomères de position de dicarbamates de diéthyloctanediol et de diallophanates de diéthyloctanediol selon l'une quelconque des revendications 1 à 4, **caractérisés en ce que** leurs chaînes de carbone en C8 présentent par rapport aux deux groupes carbamate ou groupes allophanate le motif de substitution suivant: 1,2, 1,3, 1,4, 1,5, 1,6, 1,7, 1,8, 2,3, 2,4, 2,5, 2,6, 2,7, 2,8, 3,4, 3,5, 3,6, 3,7, 3,8, 4,5, 4,6, 4,8, 5,6, 5,7, 5,8, 6,7, 6,8 ou 7,8.

6. Isomères de position de dicarbamates de diéthyloctanediol et de diallophanates de diéthyloctanediol selon la revendication 5, **caractérisés en ce que** les deux groupes carbamate ou groupes allophanate sont en position 1,5.

7. Isomères de position de dicarbamates de diéthyloctanediol et de diallophanates de diéthyloctanediol selon la revendication 6, **caractérisés en ce qu'**il s'agit de dicarbamate de 2,4-diéthyloctane-1,5-diol et de diallophanate de 2,4-diéthyloctane-1,5-diol.

8. Procédé de préparation d'isomères de position de dicarbamates de diéthyloctanediol selon l'une quelconque des revendications 1 à 7 à partir de diéthyloctanediols, caractérisé en qu'il comprend la conversion d'isomères de position de diéthyloctanediols par des carbamates d'alkyle, de cycloalkyle ou d'aryle.

9. Procédé de préparation d'isomères de position de dicarbamates de diéthyloctanediol selon l'une quelconque des revendications 1 à 7 à partir de diéthyloctanediols, caractérisé en qu'il comprend la conversion d'isomères de position de diéthyloctanediols avec du phosgène pour obtenir les chloroformiates correspondants et la conversion de ces produits intermédiaires avec de l'ammoniac et/ou des amines.

10. Procédé de préparation d'isomères de position de diallophanates de diéthyloctanediol selon l'une quelconque des revendications 1 à 7 à partir de diéthyloctanediols, caractérisé en qu'il comprend la conversion d'isomères de position de diéthyloctanediols avec des allophanates d'alkyle, de cycloalkyle ou d'aryle.

11. Procédé selon l'une quelconque des revendications 8 à 10, **caractérisé en ce que** les chaînes linéaires de carbone en C8 des isomères de position de diéthyloctanediols présentent par rapport aux deux groupes éthyle le motif de substitution suivant: 2,3, 2,4, 2,5, 2,6, 2,7, 3,4, 3,5, 3,6 ou 4,5.

12. Procédé selon la revendication 11, **caractérisé en ce que** les deux groupes éthyle sont en position 2,4.

13. Procédé selon l'une quelconque des revendications 8 à 12, **caractérisé en ce que** les chaînes de carbone en C8 des isomères de position de diéthyloctanediols présentent par rapport aux deux groupes hydroxyle le motif de substitution suivant: 1,2, 1,3, 1,4, 1,5, 1,6, 1,7, 1,8, 2,3, 2,4, 2,5, 2,6, 2,7, 2,8, 3,4, 3,5, 3,6, 3,7, 3,8, 4,5, 4,6, 4,8, 5,6, 5,7, 5,8, 6,7, 6,8 ou 7,8.

14. Procédé selon la revendication 13, **caractérisé en ce que** les deux groupes hydroxyle sont en position 1,5.

15. Procédé selon la revendication 14, **caractérisé en ce que** l'on utilise du 2,4-diéthyloctane-1,5-diol.

16. Utilisation des isomères de position de dicarbamates de diéthyloctanediol et de diallophanates de diéthyloctanediol selon l'une quelconque des revendications 1 à 7, des isomères de position de dicarbamates de diéthyloctanediol préparés à l'aide du procédé selon l'une quelconque des revendications 8, 9 ou 11 à 15 et des diallophanates de diéthyloctanediol préparés à l'aide du procédé selon l'une quelconque des revendications 10 à 15 comme modules de synthèse en chimie organique des molécules à bas poids moléculaire et à haut poids moléculaire et en chimie organique des métaux.

17. Utilisation des isomères de position de dicarbamates de diéthyloctanediol et de diallophanates de diéthyloctanediol selon l'une quelconque des revendications 1 à 7, des isomères de position de dicarbamates de diéthyloctanediol préparés à l'aide du procédé selon l'une quelconque des revendications 8, 9 ou 11 à 15 et des diallophanates de diéthyloctanediol préparés à l'aide du procédé selon l'une quelconque des revendications 10 à 15 pour la préparation d'adhésifs, de pâtes d'étanchéité et de substances de revêtement aptes à durcir thermiquement ou thermiquement et avec un rayonnement actinique.

18. Adhésifs, pâtes d'étanchéité et substances de revêtement aptes à durcir thermiquement ou thermiquement et avec un rayonnement actinique, caractérisés en qu'ils contiennent au moins l'un des dicarbamates de diéthyloctanediol et/ou au moins l'un des diallophanates de diéthyloctanediol selon l'une quelconque des revendications 1 à 7 et/ou au moins l'un des dicarbamates de diéthyloctanediol préparés à l'aide du procédé selon l'une quelconque des revendications 8, 9 ou 11 à 15 et/ou au moins l'un des diallophanates de diéthyloctanediol préparés à l'aide du procédé selon l'une quelconque des revendications 10 à 15.

19. Couches adhésives, pâtes d'étanchéité et revêtements sur et dans des substrats recouverts et non recouverts d'une couche de fond, qui peuvent être préparés à partir des adhésifs, pâtes d'étanchéité et substances de revêtement aptes à durcir thermiquement ou thermiquement et avec un rayonnement actinique selon la revendication 18.

20. Substrats recouverts et non recouverts d'une couche de fond et de couches adhésives, de pâtes d'étanchéité et/ou de revêtements selon la revendication 19.
